Europäisches Patentamt

European Patent Office

Office européen des brevets

(19)

(11) Publication number: **0 161 091 B1**

(12) **EUROPEAN PATENT SPECIFICATION**

(45) Date of publication of patent specification: **12.05.93**  (51) Int. Cl.⁵: **B41M 5/00**, B01J 13/02

(21) Application number: **85302939.5**

(22) Date of filing: **26.04.85**

(54) Fragrance–releasing pull–apart sheet.

(30) Priority: **07.05.84 US 607958**

(43) Date of publication of application:
**13.11.85 Bulletin 85/46**

(45) Publication of the grant of the patent:
**12.05.93 Bulletin 93/19**

(84) Designated Contracting States:
**DE FR GB IT**

(56) References cited:
**US–A– 4 186 743**
**US–A– 4 487 801**

(73) Proprietor: **MINNESOTA MINING AND MANU–FACTURING COMPANY**
**3M Center, P.O. Box 33427**
**St. Paul, Minnesota 55133–3427(US)**

(72) Inventor: **Sweeny, Norman P. c/o Minnesota Mining and**
**Manufacturing Co. 2501 Hudson Road P.O. Box 33427**
**St. Paul Minnesota 55133(US)**
Inventor: **Wienke, Orville F. c/o Minnesota Mining and**
**Manufacturing Co. 2501 Hudson Road P.O. Box 33427**
**St. Paul Minnesota 55133(US)**
Inventor: **Charbonneau, Jack W. c/o Minne–sota Mining and**
**Manufacturing Co. 2501 Hudson Road P.O. Box 33427**
**St. Paul Minnesota 55133(US)**

(74) Representative: **Baillie, Iain Cameron et al**
**c/o Ladas & Parry, Altheimer Eck 2**
**W–8000 München 2 (DE)**

Rank Xerox (UK) Business Services
(3.10/3.6/3.3.1)

EP 0 161 091 B1

## Description

Field of the Invention

This invention relates to microencapsulated materials, articles containing microencapsulated materials and the method of preparing such articles. In particular, the present invention relates to microencapsulated materials adhesively secured between two temporarily adhered coated paper surfaces such that upon separation of said two surfaces, the capsules rupture, releasing material contained therein.

Background of the Invention

Encapsulated materials have been used for many years in a wide variety of commercial applications. Early uses of encapsulated materials included paper coated with capsules bearing coloring material therein which could be used as a recording medium. U.S. Patent No. 3,016,308 discloses one of the early efforts using encapsulated material as the image source on recording paper. U.S. Patent Nos. 4,058,434 and 4,201,404 show other methods of application of encapsulated coloring materials on paper substrates to be used as imaging media and the like. U.S. Patent No. 3,503,783 shows microcapsules having coloring material therein which are ruptureable by the application of heat, pressure and/or radiation because of a metal coating on the surface of the capsule. These ruptureable microcapsules, in one embodiment, may be secured between a substrate and a photoconductive top coat to enable photosensitive imaging of the system.

A wide variety of processes exist by which microcapsules can be manufactured. These varied processes provide different techniques for producing capsules of varying sizes, alternative materials for the composition of the capsule shell and various different functional materials within the shell. Some of these various processes are shown in U.S. Patent Nos. 3,516,846; 3,516,941; 3,778,383; 4,087,376; 4,089,802; 4,100,103 and 4,251,386 and British Patent Specification Nos. 1,156,725; 2,041,319 and 2,048,206. A wide variety of different materials may also be used in making the capsule shells. A popular material for shell formation is the polymerization reaction product between urea and formaldehyde or melamine and formaldehyde, or the polycondensation products of monomeric or low molecular weight polymers of dimethylolurea or methylolated urea with aldehydes. A variety of capsule forming materials are disclosed, for example, in U.S. Patent Nos. 3,516,846 and 4,087,376 and U.K. Patent Specification Nos. 2,006,709 and 2,062,570.

As shown in these references, the principal utility of microencapsulated materials is in the formation of a surface coated with the microcapsules in a binder. The microcapsules are ruptured by various means to release the material contained therein. In addition to release of physically observable materials such as ink in order to form a visible image, other types of active ingredients such as odor releasing materials, bacteriostatic materials, chemically active materials and the like have been provided in this manner.

US − A − 349 4505 is a prior art disclosing means for sampling perfume fragrances. It comprises a roll of tape carried in a dispenser. The tape is a paper backing carrying a polymeric moisture barrier on which there is a coating of perfume filled microcapsules having shells of a urea − formaldehyde polymer. The dispenser is formed with a slot and associated means such that on passage of the tape therethrough the capsules are crushed on the tape coating.

US − A − 418 6743 is a prior art in which a sanitary napkin comprises a body contacting surface and a garment contacting surface coated with a pressure − sensitive adhesive. The pressure − sensitive adhesive is protected by a releasable layer having on the surface thereof that faces said pressure − sensitive adhesive coating, a microcapsule − filled adhesive. The capsules are pressure − frangible and contain a deodorant. Upon removing said protective layer some of the microcapsules fracture to release the deodorant. In this prior art there are thus two separate adhesives , one as a binder for the capsules and the other of which is pressure sensitive. The binder is such that many of the capsules therein are not at the surface of the binder and are thus not available to be gripped by the pressure sensitive adhesive so that they may be ruptured when the releasable layer is peeled away.

US − A − 4487 801 discloses a device for exposing a liquid to the atmosphere so as to enable said liquid to react, or at least partially evaporate, said device comprising

(1) at least two sheets bound by a single layer of a non − pressure sensitive adhesive composition layer.

(2) said adhesive composition layer containing micro − capsules with said liquid within the shell of the microcapsules, and

(3) said microcapsules having an average diameter between 8 and 30 micrometers, the cohesive strength of the adhesive composition layer being less than the strength of the bond between said adhesive composition and said sheets, and the tensile rupture strength of said microcapsules being less than the cohesive strength of the adhesive composition.

Summary of the Invention

The present invention comprises a device for exposing a liquid, said device comprising:

at least two surfaces of coated paper and a non−pressure sensitive adhesive composition layer extending therebetween and contacting each of said two coated paper surfaces;

said adhesive composition layer containing microcapsules with said liquid within the shells of the microcapsules;

the cohesive strength of the adhesive composition layer being less than the strength of the bond between said adhesive composition and the coated paper surfaces, the tensile rupture strength of said microcapsules being such that the cohesive failure of the adhesive results in breakage of the microcapsules;

said adhesive composition comprising a viscofier;

the microcapsules having an average diameter between 4 and 80 micrometers;

and the tensile rupture strength between said two surfaces being at least 4.0 g/cm and less than 90 g/cm at 20°C and 50 % relative humidity.

Detailed Description of the Invention

The present invention relates to an article comprising at least two surfaces, sheets or opposed faces of a folded single sheet temporarily secured by means of an adhesive layer having microcapsules dispersed therein and a viscosity increasing additive in the adhesive. Said surfaces, sheet or sheets of the present invention comprise coated paper. Generally flexible sheets of paper are preferred. Coated paper is a conventional and standard item in commerce. It is generally a fibrous sheet having a pigment−bearing resinous coating on one or both surfaces. Usually the pigment provides a white, bone or ivory coloration to the sheet. Most generally pigments producing a white coloration are used. The binder used in the resinous coating is generally colorless and/or transparent. The binder is generally a synthetic or natural organic polymeric material. Typical pigments for producing white coated paper are fine white pigment such as clay, calcium carbonate, titania, silica, zinc oxide, etc. Typical binders include latices (e.g., styrene−butadiene, butadiene−acrylonitrile, etc.), film−forming polymers (e.g., polymethylmethacrylate), and natural resins (e.g., casein, ammonium caseinate, starch, etc.). The coatings usually comprise between 65−90% by weight of pigment, preferably 70−80% by weight of pigment, and 10−35% by weight of binder, preferably 20−30% by weight of binder. Papers having both sides coated are preferred in the advertising trade.

The adhesive material for the capsules must form a bond to the coated surfaces of the sheets which is stronger than the cohesive strength of the adhesive with the capsules dispersed therein. When capsules are included within the adhesive composition, the effective adhesive strength of the adhesive tends to be reduced. Adhesives, which by themselves would cause the sheets to be damaged during separation, can be used in combination with capsules in the practice of the present invention because of lowered effective cohesive strength. The capsules in the present invention may comprise any ruptureable capsule containing an active ingredient therein. The tensile rupture strength of the capsules must be such that the cohesive failure of the adhesive results in capsule breakage. It has also been found that the size of the capsules plays a role in the usefulness of capsules within ruptureable sheets according to the practice of the present invention. Generally the capsules should have an average diameter between 6 and 50 $\mu$m and preferably between 12 and 30 $\mu$m when the capsule payload is between 80 and 90% by weight of the total capsule weight. It is highly preferred that the capsules have an average diameter between 14 and 26 $\mu$m and it is most preferred that the capsules have a diameter between 15 and 25 $\mu$m. These dimensions play a surprisingly important role in the ability to control the percentage of rupture of capsules in the practice of the present invention. With lower payloads (e.g., 70−80%), the capsules should be larger to provide the necessary rupture strength. The broadest range of capsule size under any conditions would be about 4 to 80 $\mu$m, with 8 $\mu$m capsules used with a 90−95% by weight payload. Eight to thirty $\mu$m capsules are generally preferred.

A basic relationship exists amongst the factors of peel force, adhesive coating weight and the median capsule diameter. This relationship can be expressed as $P = k \, (C_w/d^2)$, wherein P equals the peel force, $C_w$ equals the adhesive line coating weight, d equals the median diameter of the capsule, and k equals a co−efficient relating to binder and substrate properties. The peel force should be in the range of 17 to 134 grams per cm (1.5 to 12 ounces per lineal inch), preferably 17 to 89 grams per cm (1.5 to 8.0 ounces per lineal inch). The coating weight of adhesive and microcapsules should be at a coating weight of approximately one kg. for 61.44 to 163.8 m$^2$ (one pound for 300 to 800 square feet). Preferably the coating weight should be between approximately one kg for 81.9 to 133 m$^2$ (one pound for each 400 to 650 square feet). At higher coating weights, the surface of the cover sheets tend to tear, while at lower coating weights, the sheets tend to pull apart and the adhesive to paper bond tends to rupture in advance of the capsules included therein. The capsules should form between 20 and 90 percent by volume of the total adhesive composition, and preferably between 50 and 85 percent of the total adhesive composition volume. If certain microcapsule shell materials are used, such as gelatin, the capsule may comprise as much as 100% of the adhesive compositions.

Even with these considerations being met, the coated surfaces of coated paper could not be used successfully as the adhesively bonded surface in the manufacture of two faces or sheets bearing releasable microencapsulated materials between them. It was found that the bond of the conventional adhesives to the coating on the paper and the distribution of the adhesive was not uniform. The adhesive bond between the paper coating and the capsule – bearing adhesive would fail before the cohesive strength of the capsule – bearing adhesive would, fibers would either be pulled from the paper or adhesive would fail without rupture of large percentages of capsules. It was difficult to satisfactorily adhere the capsule – bearing adhesive to the coated paper surface, with irregular appearance and non – uniform bond strength developing. Addition – ally, the adhesive tended to soak into the paper, causing microcapsules to be insufficiently bonded to the surface or the bond between the sheets to be too weak.

It is preferred that the rupture strength between the sheets excedes 8.0g/cm and is less than 80g/cm and most preferably excedes 16g/cm and is less than 75g/cm. The minimum strength at this ambient condition (i.e., 20˚C and 50% R.H.) is necessary to keep the sheets from falling apart from forces incurred during handling. This problem has frequently occurred in magazine inserts where coated paper was used. The maximum limit on the rupture strength is necessary to keep the paper from tearing (termed fiber pull or fiber rupture) before the adhesive and capsules rupture. This would prevent release of the liquid from the capsules.

It is also desirable to have the construction resist the effects of variable ambient conditions. Certain products presently used on uncoated paper stock work in ambient conditions but fail in transit or on storage as the temperature and humidity change. Given the fact that some of these compositions fail at even standard conditions (20˚C and 50% R.H.), they tend to fail worse at more extreme conditions such as 30˚C and 80% R.H. or on dry conditions. For example, some binders or capsules are dehydrated by storage in heated warehouses during the winter and become so fragile that simple handling will rupture them. Complaints have been made by purchasers of magazines that all of the various odors in inserts are being released prior to usage of the magazine. The entire magazine tends to have a strong composite odor of many scents rather than being able to provide distinct individual scents. It is therefore desirable that rupture strength excede 4.0g/cm after storage at 49˚C (120˚F) and less than 1% R.H. for seventy – two hours. This test would be performed by storage in an oven, removal to a neutral environment (e.g., sealed bag or jar) until the article is at room temperature, and then measuring the rupture strength. It is preferred that the rupture strength is at least 8.0g/cm and most preferred that the rupture strength is at least 16g/cm under those conditions. The article must still display a rupture strength between 4 and 90g/cm at 20˚C and 50% R.H.

A number of methods have been found which enable these conditions to be met according to the present invention. The use of viscosity increasing agents in the binder provides a more even coating and one that ruptures before fiber pull begins. The use of additional coatings over the coated paper which contain polymers different from the binder of the adhesive layer and which do not form a solution or chemically bond to the binder of the adhesive layer provides a useful article according to the present invention. The use of larger size capsules tends to weaken the cohesive strength of the adhesive composite and prevent fiber pull. The use of capsules which are not moisture sensitive in combination with these large capsules (i.e., greater than 30 μm and up to 95 μm) provides a useful adhesive layer. Higher capsule – to – binder ratios reduce the cohesive strength of the adhesive, as does the addition of non – viscosity enhancing particulate fillers.

According to the present invention, it has been found that the method of the addition of viscosity increasers (viscofiers), for reasons unknown, solved all of the above – identified problems in the use of coated paper base. The use of viscofiers also reduced the criticality of proportions of materials and provided increased coating and manufacturing latitude. Viscosity enhancers or viscosity increasing agents are well known in the art. Any material which when present in the coating solution in an amount not greater than 10% by weight increases the viscosity by at least 5% is a viscofier according to the present invention. Preferably viscosity is increased by at least 20%. They are either inorganic particulate materials (e.g., silica, amorphous silica, bentonite clay, montmorillonite clay, etc.) or organic particulate or soluble materials (e.g.,

water softenable acrylic particles, water swellable poly(methyl – methacrylate), water soluble or organic solvent soluble polymers, etc.). The inorganic particles tend to be preferred. The viscofiers have been found to be necessary in dry weight proportions of the adhesive mix in amounts of from 0.25 to 12% by weight, preferably from 5 to 12% by weight. In general, the weight proportions of materials in the dried adhesive layers according to the present invention are generally as follows:

| Microcapsules | 21 – 80% |
| Adhesive | 19.75 – 70% |
| Viscosity Enhancers | 0.25 – 12% |

Other optional ingredients such as surfactants, coating aids and the like may be present. Preferred proportions of these ingredients are:

| Microcapsules | 44.5 – 80% |
| Adhesive | 19.5 – 55% |
| Viscosity Enhancers | 0.5 – 10% |

The ability to use coated paper in the manufacture of these articles is important because that material is the standard printing medium of the trade. Those papers enable the highest quality printings to be made in combination with the releasable materials of the present invention.

The adhesive (with microcapsules) may be applied between two separate sheets in either a continuous or discontinuous pattern. It is usually desirable to leave at least some portion of at least one outer edge of the sheets unbonded so as to provide an area where separation can be easily started. A single sheet may be folded so as to form two facing sheets joined along one edge. The adhesive may be applied on the interior area adjacent the fold. This provides a folded article that can be readily opened, rupturing the capsules, yet leaves a single artifact rather than two sheets after use.

It is preferred that the capsule – bearing adhesive coated inside portion of the single sheets (e.g., from the fold to the end of the adhesive) constitute from 5 to 40% of the surface area of the sheets. In two sheet constructions, 10 to 95 percent adhesive coverage is used. Some uses may allow for only a single corner to be uncoated so as to provide a starting point for the separation of the sheets, but the 5 to 40% range is preferred with 10 to 30% more preferred in two sheet constructions.

Any class of adhesives including but not limited to polyurethanes, polyacrylates, polyvinyl resins (e.g., polyvinyl alcohol, polyvinyl chloride), polyamides, polyesters, polyolefins, starches, gum arabic, gelatin and the like may be readily used in the practice of the present invention. Washing of the capsules before mixing them with the adhesive tends to provide more consistency in their properties by removing low molecular weight, unreacted materials.

In effect, to best practice the present invention it is desirable that certain properties within the article have relative values for each of the materials used. The cohesive strength of the sheet material should exceed the adhesive strength between the binder and the sheet. The adhesive strength of the binder to the sheet should exceed the cohesive strength of the binder and capsules therein. The cohesive strength of the binder should exceed the tensile rupture limits of the capsules.

As previously noted, the size of the capsules has an important effect upon the practice of the present invention. With capsules less than 8 $\mu$m, there tends to be less rupturing of the capsules as to prevent the useful and efficient release of materials. Above 30 $\mu$m, the particles are so large that they are more readily burst by handling of the sheets and manufacturing procedures. Furthermore, with the large size particles it is extremely difficult to control bursting upon separation of the sheets because of increased effects upon adhesive and cohesive properties of materials in contact with the capsules. The preferred ranges of 8 to 30 and 15 to 25 $\mu$m is important to the practice of the present invention. Within these limits, rupture in excess of 50 percent of the capsules can be easily obtained. Rupture in excess of 80 percent of the capsules can often be accomplished in the practice of the present invention within those limits.

The capsules may contain a wide variety of active materials therein. The least useful of materials to be included therein would be coloring agents since separation of the sheets would generally produce uniform coloration rather than a distinct image. The most preferred types of ingredients would be fragrant materials (such as essences and perfumes) or materials which provide chemically active vapors or liquids (e.g., bacteriostats or deodorants) to be wiped on or transferred to another surface. These may or may not also be colored. For example, a testing kit for the presence of chemical vapors could be produced by providing material within the capsules which would react in the vapor phase with the material for which a leak is being investigated. By separating the sheet, rupturing the capsules and exposing the vapor test material, a color forming reaction in the air or on the sheet could be readily observable. Another particularly useful format would be to include the microcapsules within a water – remoistenable adhesive and to use the mixture as the binding adhesive for novelty envelopes. For example, the microcapsules could contain the aromatic essence of baby oil, cake or pizza for invitation envelopes for a baby shower, wedding (or birthday party), or general party, respectively. The sides of the sheets with the capsule – bearing adhesive thereon are preferably printed under the adhesive or adjacent the adhesive.

This invention may be practiced with a number of various modifications that provide new and useful articles and processes. For example, the adhesive composition with capsules may be associated with various printed formats to form novelty items. The exterior sheets or exposed inner face of the sheets may have questions or stories or rhymes, and under the adhesive may be a printed picture answering the question, depicting the story or completing the rhyme, with the released fragrance emphasizing the picture further.

The capsule bearing adhesive layer in the construction of the present invention may also be used for a security device. In an article such as a coupon, lottery ticket or gaming card, the important display could be located under the adhesive. Once the article had been opened and the fragrance released, any subsequent recipient would be aware of its prior use and could be apprised of the possibility of tampering. The adhesive being non – pressure sensitive, it is not repositionable, the sheets are not easily rebonded, and there would be no release of fragrance if the sheets were rebonded with additional non – fragranced adhesive and reopened. The absence or reduced level of fragrance would indicate that the article had been tampered with.

These and other aspects of the present invention will be shown in the following examples.

Example 1

An oil having the aroma of roses was encapsulated in a urea – formaldehyde resin made according to the process of Example 20 of U.S. Patent No. 3,516,941. The capsules had an average diameter of about 17 micrometers and an estimated payload of 85% by weight (ratio of oil to total capsule weight).

A coating formulation was prepared comprising 58.30 parts capsules, 31.90 parts polyvinyl alcohol, 0.9 parts glycerine (plasticizer) and 8.90 parts Syloid® amorphous silica viscosity enhancer in a water slurry. This formulation was coated at 1 kg. per 88.74 $m^2$ (3.0 lbs. per 1300 sq. ft.) (dry weight) onto double – side coated paper base stock. The coating was made in a stripe down the middle of the paper and the paper folded sharply around the stripe after coating. The coated and folded paper was air dried at ambient conditions for two days.

Sections of the coated paper were cut to provide a folded sheet with a 20% portion of the paper extending from the fold coated with adhesive and capsules. The edges of the sheets were grasped by hand and pulled open sharply. There was a burst of rose aroma after the interior adhesive strip was ruptured.

The same composition, without the addition of the amorphous silica provided a functional article but of much poorer quality. The adhesive coating was uneven, microcapsule agglomerated, and a lower percent – age of capsules were therefore ruptured during use.

Examples 2 – 3

Example 1 was twice repeated, replacing the silica viscofier with equal weight amounts of 1) a water soluble carboxyvinyl polymer viscofier (Carbopol® 691) and 2) sodium alginate, a water soluble viscofier. The coatings appeared smooth and uniform. Upon separation of the adhesively secured faces, a high percentage of the capsules were ruptured.

Examples 4 – 9

Example 1 was repeated without the use of the viscosity enhancing agent. The rupture strength of the adhesive then exceeded 45g/cm and the paper was torn without significant rupture of the adhesive and capsules.

The same adhesive composition was then used on five sheets of coated paper which were first primed with five different precoats, 1) high molecular weight poly(vinyl alcohol), 2) low molecular weight poly(vinyl alcohol), 3) poly(vinyl pyrrolidone), 4) an acrylic resin, and 5) a polyurethane resin. The first four coatings blended with the adhesive coating or chemically bonded thereto, raising the rupture strength to more than

6

45g/cm. Only the polyurethane coating which neither bonded nor dissolved in the adhesive binder provided a useful coating. The bond strength was about 4g/cm with that coating at 20°C and 50% R.H.

The binder composition of Example 1 without the viscosity enhancing agent was used with 85 μm capsules instead of the 20 μm capsules. This coating worked well, but was not preferred because the large capsules could be broken during handling without rupture of the adhesive. The bond strength to the coated paper was between 30 and 50g/cm.

Example 10

The binder composition of Example 1 without the viscosity enhancing agent was used with 85 μm capsules instead of the 20 μm capsules. This coating worked well, but was not preferred because the large capsules could be broken during handling without rupture of the adhesive. The bond strength to the coated paper was between 30 and 50g/cm.

Example 11

The binder composition of Example 1 without the viscosity enhancing agent was used with 20 μm capsules having an 88% payload instead of an 85% payload. This was done by using less resin during the formation of the microcapsules and produced a thinner shell article (hence a greater payload). The thinner shell produced a weaker capsule, and therefore a weaker adhesive. The rupture strength was still in the high end of the acceptable range at about 80g/cm.

Example 12

The binder composition of Example 1 without the viscosity enhancing agent was used with the capsules of that example and additionally 5% by dry weight of 25 μm silica particles. These particles reduced the cohesive strength of the adhesive layer to about 40g/cm.

**Claims**

1. A device for exposing a liquid, said device comprising:
   at least two surfaces of coated paper and a non−pressure sensitive adhesive composition layer extending therebetween and contacting each of said two coated paper surfaces;
   said adhesive composition layer containing microcapsules with said liquid within the shells of the microcapsules;
   the cohesive strength of the adhesive composition layer being less than the strength of the bond between said adhesive composition and the coated paper surfaces, the tensile rupture strength of said microcapsules being such that the cohesive failure of the adhesive results in breakage of the microcapsules;
   **characterized in that**
   said adhesive composition comprises a viscofier;
   the microcapsules have an average diameter between 4 and 80 micrometers;
   and the tensile rupture strength between said two surfaces is at least 4.0 g/cm and less than 90 g/cm at 20°C and 50 % relative humidity.

2. The device of Claim 1 wherein the viscofier is one of the group comprising: silica, in particular amorphous silica; bentonite clay, in particular montmorillonite clay.

3. The device of Claim 1 wherein the viscofier one of the group comprising: equal amounts of a water soluble carboxyvinyl polymer and a water soluble sodium alginate; a water softenable acrylic; a water swellable polymethylmethacrylate; a water soluble or organic solvent soluble polymer.

4. The device of Claim 1 wherein said coated papers are flexible and said viscofier comprises from 0.25 to 12 % by dry weight of said adhesive composition layer.

5. The device of Claim 4 wherein said coated paper is coated on both faces with a resinous binder and pigment.

7

**6.** The device of Claim 1 wherein said microcapsules have an average diameter between 8 and 30 micrometers.

**7.** The device of Claim 1 wherein said microcapsules comprise gelatin and are between 21 % and 100 % weight of said adhesive composition, said adhesive composition comprising a binder that is up to 78.75% by weight of the dried adhesive layer and said viscofier comprises between 0.25 and 12% by weight of the dried adhesive layer.

**8.** The device of Claims 5, 6 or 7 wherein said microcapsules comprise between 50 and 85 % by volume of said adhesive composition.

**9.** The device of Claim 1 wherein said liquid is an odor releasing material and the shells of said microcapsules comprises a urea – formaldehyde resin.

**10.** The device of Claims 1, 4, 5 or 6 wherein said liquid is an odor releasing material.

**11.** The device of Claim 1 wherein a polymeric coating is present over said coated paper surface which is not dissolved by or chemically bonded to said adhesive composition.

**Patentansprüche**

**1.** Vorrichtung zum Exponieren einer Flüssigkeit, umfassend:
mindestens zwei Oberflächen von beschichtetem Papier und einer Schicht einer nichtdruckemp – findlichen Kleberzusammensetzung, die sich dazwischen befindet und mit jeder der zwei beschichteten Papieroberflächen in Kontakt ist;
die Schicht der Kleberzusammensetzung, welche Mikrokapseln mit der Flüssigkeit im Inneren der Umhüllungen der Mikrokapseln enthält;
die Kohäsionsfestigkeit der Schicht der Kleberzusammensetzung kleiner ist als die Haftfestigkeit zwischen der Kleberzusammensetzung und den beschichteten Papieroberflächen und die Zerreiß – Berstfestigkeit der Mikrokapseln so groß ist, daß der Kohäsionsbruch des Klebers zum Bruch der Mikrokapseln führt,
**dadurch gekennzeichnet, da***ß*
die Kleberzusammensetzung ein Dickungsmittel aufweist;
die Mikrokapseln einen mittleren Durchmesser zwischen 4 und 80 Mikrometer haben und
die Zerreiß – Berstfestigkeit zwischen den zwei Oberflächen bei 20 ˚C und 50 % relativer Luftfeuchtigkeit mindestens 4,0 g/cm und weniger als 90 g/cm beträgt.

**2.** Vorrichtung nach Anspruch 1, bei welcher das Dickungsmittel zu einer Gruppe gehört, umfassend: Silica, insbesondere amorphes Silica, Bentonit – Ton, insbesondere Montmorillonit – Ton.

**3.** Vorrichtung nach Anspruch 1, bei welcher das Dickungsmittel zu einer der Gruppe gehört, umfassend: gleiche Mengen eines wasserlöslichen Carboxyvinylpolymers und ein wasserlösliches Natriumalginat, ein Acrylharz, das in Wasser erweichen kann, ein in Wasser quellfähiges Polymethylmethacrylat, ein in Wasser oder in einem organischen Lösungsmittel lösliches Polymer.

**4.** Vorrichtung nach Anspruch 1, bei welcher die beschichteten Papiere flexibel sind und das Dickungs – mittel von 0,25 bis 12 % Trockenmasse der genannten Schicht der Kleberzusammensetzung aufweist.

**5.** Vorrichtung nach Anspruch 4, bei welcher das beschichtete Papier auf beiden Seiten mit einem harzartigen Bindemittel und Pigment beschichtet ist.

**6.** Vorrichtung nach Anspruch 1, bei welcher die Mikrokapseln einen mittleren Durchmesser zwischen 8 und 30 Mikrometer haben.

**7.** Vorrichtung nach Anspruch 1, bei welcher die Mikrokapseln Gelatine aufweisen und zwischen 21 Gewichtsprozent und 100 Gewichtsprozent der Kleberzusammensetzung ausmachen, wobei die Kle – berzusammensetzung ein Bindemittel mit bis zu 78,75 Gewichtsprozent der getrockneten Kleberschicht und das Dickungsmittel zwischen 0,25 und 12 Gewichtsprozent der getrockneten Kleberschicht umfas –

sen.

**8.** Vorrichtung nach den Ansprüchen 5, 6 oder 7, bei welcher die Mikrokapseln zwischen 50 und 85 Volumenprozent der Kleberzusammensetzung aufweisen.

**9.** Vorrichtung nach Anspruch 1, bei welcher die Flüssigkeit eine Substanz ist, die einen Geruch freisetzt, und die Hüllen der Mikrokapseln ein Harnstoff − Formaldehydharz aufweisen.

**10.** Vorrichtung nach Anspruch 1, 4, 5 oder 6, bei welcher die Flüssigkeit eines Substanz ist, die einen Geruch freisetzt.

**11.** Vorrichtung nach Anspruch 1, bei welcher eine polymere Beschichtung auf der beschichteten Papier − oberfläche vorhanden ist, welche von der Kleberzusammensetzung nicht aufgelöst oder chemisch von ihr gebunden wird.

**Revendications**

**1.** Dispositif pour exposer un liquide, ledit dispositif comprenant :
au moins deux surfaces de papier couché et une couche de composition adhésive non sensible à la pression située entre ces deux surfaces et étant en contact avec chacune desdites deux surfaces de papier couché;
ladite couche de composition adhésive contenant des microcapsules, ledit liquide étant contenu dans les enveloppes des microcapsules;
la force de cohésion de la couche de composition adhésive étant inférieure à la force de liaison entre ladite composition adhésive et les surfaces de papier, la force de rupture sous traction desdites microcapsules étant telle que le défaut de cohésion de l'adhésif entraîne une rupture des microcapsu − les;
caractérisé en ce que
ladite composition adhésive contient un agent de viscosité;
les microcapsules ont un diamètre moyen compris entre 4 et 80 micromètres;
et la force de rupture sous traction entre lesdites deux surfaces est d'au moins 4,0 g/cm et inférieure à 90 g/cm à 20˚C et 50 % d'humidité relative.

**2.** Dispositif salon la revendication 1, dans lequel l'agent de viscosité fait partie du groupe comportant des silices, en particulier la silice amorphe; l'argile de bentonite, en particulier l'argile de montmorillonite.

**3.** Dispositif selon la revendication 1, dans lequel l'agent de viscosité fait partie du groupe comportant des quantités égales de polymère carboxyvinyle hydrosoluble et de l'alginate de sodium soluble dans l'eau; un acryclique ramolissable dans l'eau; un polymétylméthacrylate gonflable dans 1 eau; un polymère soluble dans un solvant organique ou soluble dans l'eau.

**4.** Dispositif salon la revendication 1 dans lequel lesdits papiers couchés sont souples et ledit agent de viscosité comprend de 0,25 à 12 % en poids sec de ladite couche de composition adhésive.

**5.** Dispositif selon la revendication 4 dans lequel ledit papier couché est couché sur ses deux faces avec un liant de résine et un pigment.

**6.** Dispositif selon la revendication 1 dans lequel lesdites microcapsulea ont un diamètre moyen compris entre 8 et 30 micromètres.

**7.** Dispositif selon la revendication 1 dans lequel lesdites microcapsules contiennent de la gélatine et représentent entre 21 % et 100 % en poids de ladite composition adhésive, ladite composition adhésive comportant un liant représentant 78,75 % en poids de la couche adhésive séchée et ledit agent de viscosité comprenant entre 0,25 et 12 % en poids de la couche adhésive séchée.

**8.** Dispositif selon les revendications 5, 6 ou 7 dans lequel lesdites microcapsules comprennent entre 50 et 85 % en volume de ladite composition adhésive.

**9.** Dispositif selon la revendication 1 dans lequel ledit liquide est une substance libérant une odeur et les enveloppes desdites microcapsules contiennent une résine urée – formaldéhyde.

**10.** Dispositif selon les revendications 4, 5 ou 6 dans lequel ledit liquidé est une substance libérant une odeur.

**11.** Dispositif selon la revendication 1 dans lequel ladite surface de papier couché est recouverte d'un couchage polymère qui n'est pas dissous par ou chimiquement lié à ladite composition adhésive.